# EUROPEAN PATENT APPLICATION

(11) **EP 3 202 895 A1**
(43) Date of publication of application: **09.08.2017**
(21) Application number: 17162282.2
(22) Date of filing: 01.03.2005
(51) Int. Cl.: C12N 5/0783, A61K 35/14

(54) **A COMPOSITION FOR USE IN CELL THERAPY AND METHODS FOR ITS PREPARATION**

(30) Priority: 01.03.2004 US 549032 P
(62) Divisional of application: 05724065.7
(71) Applicant: Immunovative Therapies, Ltd., 20179 Misgav (IL)
(72) Inventor: HAR-NOY, Michael, Jerusalem (IL)
(74) Representative: Richards, Michèle E.

(57) **Abstract**

A composition is described that comprises a treatment effective amount of cells, of which a portion are T-cells, wherein the T-cells have been harvested from cell culture and are thereby removed from activating stimuli in the culture and the harvested T-cells are formulated for infusion by a method comprising (1) contacting the T-cells with one or more agents that ligate a cell surface moiety on at least a portion of the T-cells, (2) cross-linking the one or more agents with biodegradable nanospheres or microspheres coated with a material capable of cross-linking the one or more agents, (3) suspending the T-cells, the one or more agents and the coated biodegradable nanospheres or microspheres in an infusion medium, and (4) packaging in a suitable container, wherein the T-cells are in an activated state in the medium, wherein the one or more agents are mouse-derived monoclonal antibodies, or fragments or genetically engineered derivatives thereof, and the material coating the nanospheres or microspheres is goat or sheep anti-mouse polyclonal antibodies. Methods for the preparation of the composition are also described.

## Description

### FIELD OF INVENTION

This invention relates to methods for formulating ex-vivo prepared T-cells for infusion.

### BACKGROUND OF THE INVENTION

Cell therapy methods have been developed in order to enhance the host immune response to tumors, viruses and bacterial pathogens. Cell therapy methods often involve the ex-vivo activation and expansion of T-cells. Examples of these type of treatments include the use of tumor infiltrating lymphocyte (TIL) cells (see U.S. Patent No. 5,126,132 issued to Rosenberg), cytotoxic T-cells (see U.S. Patent No. 6,255,073 issued to Cai, et al.; and U.S. Patent No. 5,846,827 issued to Celis, et al.), expanded tumor draining lymph node cells (see U.S. Patent No. 6,251,385 issued to Terman), and various other lymphocyte preparations (see U.S. Patent No. 6,194,207 issued to Bell, et al.; U.S. Patent No. 5,443,983 issued to Ochoa, et al.; U.S. Patent No 6,040,177 issued to Riddell, et al.; U.S. Patent No. 5,766,920 issued to Babbitt, et al.).

For maximum effectiveness of T-cells in cell therapy protocols, the ex vivo activated T-cell population should be in a state of maximal activation upon infusion. Efforts for developing improved methods for producing more effective T-cells for use in cell therapy protocols have focused on the ex-vivo activation methods. However, ex-vivo activated cells need to be harvested and administered to patients to have a therapeutic effect. The harvesting of the T-cells removes them from the activating stimuli available in the ex-vivo cultures. Therefore, the longer the time from harvest to infusion, the lower the quality of the T-cells.

There is a need to develop T-cell formulations for infusion that maintain the cells in a state that can maximally orchestrate an immune response to cancer, infectious diseases and other disease states at both the time of infusion and while circulating in the blood. Efforts to maintain the activation state of T-cells at the time of infusion have most commonly involved the formulation of the T-cells with exogenous IL-2. Systemic IL-2 administration to patients has also been used to maintain the activation state of T-cells post-infusion. However, exogenous IL-2 administration is extremely toxic to patients and has not resulted in significant benefit to patients undergoing T-cell infusions.

### SUMMARY OF THE INVENTION

This invention includes ex-vivo prepared T-cells which are harvested from cell culture conditions and formulated in media suitable for infusion. The method of formulation involves: (1) labeling the cells with one or more agents which have reactivity for T-cell surface moieties capable of delivering activation signals upon cross-linking; (2) mixing the labeled cells with biodegradable nanospheres or microspheres coated with a material capable of cross-linking the agents attached to the T-cell surface moieties; (3) suspending the mixture of labeled T-cells and coated biodegradable cross-linking spheres in a medium suitable for infusion; (4) packaging the mixture in a suitable container, such as a syringe or an IV infusion bag; and (5) parenteral administration of said mixture to a patient.

Alternatively, the invention can be accomplished with a formulation method which involves: (1) mixing a population of T-cells with biodegradable nanospheres or microspheres coated with a first material and one or more second materials, whereas the first material binds the second materials and the second materials have reactivity for surface moieties on the T-cells, and whereas the interaction of the second materials with the T-cells causes the activation of the T-cells; (2) suspending the mixture of T-cells and the biodegradable spheres in a medium suitable for infusion; and (3) packaging the mixture in a suitable container, such as a syringe or an IV infusion bag; and (4) parenteral administration of the mixture to a patient.

In practice of the present invention, the T-cells intended for infusion are first harvested from the ex-vivo cell culture environment. The harvested cells are then either immediately formulated for infusion, cryopreserved for later formulation, or shipped to the patient location for formulation.

Upon formulation by the method of the present invention, all the T-cells for infusion will be maximally activated in a non-toxic delivery vehicle.

For the purposes of the present invention, all references to T-cells includes a population of cells with at least a portion of the cells containing T-cells. T-cells are cells which express TCR, including α/β and γ/δ TCRs. T-cells include all cells which express CD3, including T-cell subsets which also express CD4 and CD8. T-cells include both naïve and memory cells and effector cells such as CTL. T-cells also include regulatory cells such as Th1, Tc1, Th2, Tc2, Th3, Treg, and Tr1 cells. T-cells also include NKT-cells and similar unique classes of the T-cell lineage.

### PREFERRED EMBODIMENTS

The biodegradable spheres of the present invention are preferably manufactured from aliphatic polyesters, such as poly(lactic acid) (PLA), poly(glycolic acid) (PGA), copolymers of PLA and PGA (PLGA) or poly(carprolactone) (PCL), and polyanhydrides. The spheres are produced into small particle sizes of 0.1 to 500 microns, preferably less than 10 microns and most preferably less than 1 micron. Microspheres of this size range are capable of direct injection into the body by conventional methods. It is preferred that the coated spheres be designed to degrade in physiological fluids within 7 days, more preferably within 3 days.

The preferred first material for coating on the biodegradable spheres is polyclonal goat (or sheep) anti-mouse polyclonal antibodies. By way of example, this preferred first material can be used to cross-link mouse-derived monoclonal antibodies, or fragments or genetically engineered derivatives thereof, that have specificity for T-cell surface moieties. Thus, for example, the mixing of goat anti-mouse coated microspheres (or nanospheres) with human T-cells labeled with mouse anti-human CD3 and mouse anti-human CD28 mAbs will cause the cross-linking of the mouse mAbs on the human T-cells through the binding of the goat anti-mouse polyclonal antibody with the mouse mAbs. The cross-linking of the mAbs causes the activation of the T-cells. Alternatively, the anti-human CD3 and anti-CD28 can also be first bound, preferably in a 50/50 ratio, on the goat (or sheep) polyclonal antibody coated spheres and mixed with the T-cells. It will be obvious to those skilled in the art that many combinations of first materials and second materials can be used to accomplish the objective of cross-linking second agents attached to T-cell surface moieties in order to initiate signal transduction and activation of T-cells.

The mixture of T-cells with cross-linked surface moieties is suspended in infusion medium (e.g., isotonic solutions such as normal saline, 5% dextrose, Plasma-Lyte (Baxter) or Normasol (Abbott). In some embodiments, the infusion medium is supplemented with 0.5%-10% human serum albumen (HSA).

The mixture is preferably adjusted to a final T-cell concentration of between 1x 10⁷ to 1 x 10⁸ cells per ml of infusion media. In a preferred embodiment, 10⁹ T-cells are formulated in 100 ml of infusion media. The formulation is then packaged in one or more containers, such as syringes, plastic pouches, or plastic bottles.

### STATEMENTS OF INVENTION

1. A composition comprising a treatment effective amount of cells, of which a portion are T-cells, whereby said T-cells are contacted with one or more first agents that ligate a cell surface moiety on at least a portion of the T-cells, and whereby the first agents are cross-linked by a second agent attached to a biodegradable support, and whereby said T-cells and biodegradable supports are suspended in a medium suitable for parenteral infusion and packaged in a suitable container.
2. The composition of paragraph 1 whereby the container is collapsible comprising opposing walls of flexible material and a flexible tube protruding from the container.
3. The composition of paragraph 1 whereby the container is a syringe.
4. A method for preparing a treatment effective amount of cells, of which a portion are T-cells, the method comprising:
   labeling the cells with one or more agents which have reactivity for T-cell surface moieties capable of delivering activation signals upon cross-linking;
   mixing the labeled cells with biodegradable nanospheres or microspheres coated with a material capable of cross-linking the agents attached to the T-cell surface moieties;
   suspending the mixture of labeled T-cells and coated biodegradable cross-linking spheres in a medium suitable for infusion; and
   packaging the mixture in a suitable container.
5. The method of paragraph 4 wherein the container is a syringe or an IV infusion bag.
6. The method of paragraph 4 wherein the container is collapsible.
7. The method of paragraph 6 wherein the container includes opposing walls of flexible material and an exit tube.
8. A method for preparing a treatment effective amount of cells, of which a portion are T-cells, the method comprising:
   mixing a population of T-cells with biodegradable nanospheres or microspheres coated with a first material and one or more second materials;
   whereas the first material binds the second materials, and the second materials have reactivity for surface moieties on the T-cells, and whereas the interaction of the second materials with the T-cells causes the activation of the T-cells;
   suspending the mixture of T-cells and the biodegradable spheres in a medium suitable for infusion; and
   packaging the mixture in a container.
9. The method of paragraph 8 wherein the container is a syringe or an IV infusion bag.
10. The method of paragraph 8 wherein the container is collapsible.
11. The method of paragraph 10 wherein the container includes opposing walls of flexible material and an exit tube.
12. The method of paragraph 8 wherein the package mixture of the T-cell concentration is at least 1 x 10⁷ cells per ml of infusion media.

Although the present invention has been described with reference to preferred embodiments, workers skilled in the art will recognize that changes may be made in form and detail without departing from the spirit and scope of the invention.

## Claims

1. A composition comprising a treatment effective amount of cells, of which a portion are T-cells, wherein the T-cells have been harvested from cell culture and are thereby removed from activating stimuli in the culture and the harvested T-cells are formulated for infusion by a method comprising (1) contacting the T-cells with one or more agents that ligate a cell surface moiety on at least a portion of the T-cells, (2) cross-linking the one or more agents with biodegradable nanospheres or microspheres coated with a material capable of cross-linking the one or more agents, (3) suspending the T-cells, the one or more agents and the coated biodegradable nanospheres or microspheres in an infusion medium, and (4) packaging in a suitable container, wherein the T-cells are in an activated state in the medium, wherein the one or more agents are mouse-derived monoclonal antibodies, or fragments or genetically engineered derivatives thereof, and the material coating the nanospheres or microspheres is goat or sheep anti-mouse polyclonal antibodies.

2. The composition of claim 1, wherein the T-cells are human T-cells and the monoclonal antibodies are mouse anti-human CD3 and mouse anti-human CD28 monoclonal antibodies.

3. The composition of claim 1 or claim 2, wherein the medium is suitable for parenteral infusion.

4. The composition of any one of claims 1 to 3, whereby the container is collapsible.

5. The composition of any one of claims 1 to 4, whereby the container has a syringe portion.

6. A method for preparing a treatment effective amount of cells, of which a portion are T-cells, wherein the T-cells have been harvested from cell culture and are thereby removed from activating stimuli in the culture, the method comprising formulating the harvested T-cells for infusion by a method comprising: (1) labeling the harvested T-cells with one or more agents which have reactivity for T-cell surface moieties capable of delivering activation signals upon cross-linking; (2) mixing the labeled cells with biodegradable nanospheres or microspheres coated with a material capable of cross-linking the agents attached to the T-cell surface moieties; (3) suspending the mixture of labeled T-cells and coated biodegradable cross-linking spheres in an infusion medium, whereby the T-cells are in an activated state; and (4) packaging the mixture in a suitable container, wherein the one or more agents are mouse-derived monoclonal antibodies, or fragments or genetically engineered derivatives thereof, and the material coating the nanospheres or microspheres is goat or sheep anti-mouse polyclonal antibodies.

7. A method for preparing a treatment effective amount of cells, of which a portion are T-cells, wherein the T-cells have been harvested from cell culture and are thereby removed from activating stimuli in the culture, the method comprising formulating the harvested T-cells for infusion by a method comprising: (1) mixing a population of the harvested T-cells with biodegradable nanospheres or microspheres coated with a first material and one or more second materials; wherein the first material cross-links the second materials, and the second materials have reactivity for surface moieties on the T-cells, and wherein the interaction of the second materials with the T-cells causes the activation of the T- cells; (2) suspending the mixture of T-cells and the biodegradable spheres in an infusion medium, whereby the T-cells are in an activated state; and (3) packaging the mixture in a container, wherein the one or more agents are mouse-derived monoclonal antibodies, or fragments or genetically engineered derivatives thereof, and the material coating the nanospheres or microspheres is goat or sheep anti-mouse polyclonal antibodies.

8. The methods of claim 6 or claim 7, wherein the T-cells are human T-cells and the monoclonal antibodies are mouse anti-human CD3 and mouse anti-human CD28 monoclonal antibodies.

9. The method of any one of claims 6 to 8 wherein the container is a syringe or an IV infusion bag.

10. The method of any one of claims 6 to 9 wherein the container is collapsible.

11. The method of any one of claims 6 to 10 wherein the container includes opposing walls of flexible material and an exit tube.

12. The method of any one of claims 6 to 11 wherein the package mixture of the T-cell concentration is at least 1 x 10⁷ cells per ml of infusion media.
